# EUROPEAN PATENT APPLICATION

(11) **EP 1 118 341 A1**
(43) Date of publication of application: **25.07.2001**
(21) Application number: 00100259.1
(22) Date of filing: 18.01.2000
(51) Int. Cl.: A61L 15/36, A61F 13/15, B32B 9/00, A61F 13/14

(54) **Breast pads for nursing mothers comprising lactic-acid producing micro-organisms**

(71) Applicant: THE PROCTER & GAMBLE COMPANY, Cincinnati, Ohio 45202 (US)
(72) Inventor: Di Cintio, Achille, 65126 Pescara (IT); Pesce, Antonella, 65120 Pescara (IT); Toro, Carlo, 65012 Cepagatti (Pescara) (IT); Carlucci, Giovanni, 66100 Chieti (IT)
(74) Representative: Kremer, Véronique Marie Joséphine

(57) **Abstract**

The present invention relates to breast pads for nursing mothers which comprise lactic acid producing micro-organisms, preferably *L. sporogenes.* These breast pads exhibit effective odour control and reduce the risk of skin infections and/or skin irritations while being safe to babies.

## Description

### Field of the Invention

This invention relates to breast pads for nursing mothers and more particularly to breast pads designated to reduce or even prevent malodours associated with milk discharge while minimising the occurrence of skin irritation and/or skin infection.

### Background of the Invention

In the recent years women have become increasingly active outside the home and in various work environments. Maternity leaves from employment following the birth of a child are commonly quite short. At the same time, the popularity of breast feeding babies has experienced a resurgence. In order to prevent staining of clothing and embarrassment caused by leaking of fluid, nursing mothers employ pads to absorb leaking fluid. Such pads are commonly placed inside a bra. The pads employed can be flat pads or may be shaped pads particularly adapted to conform to the shape of a breast.

Such pads although providing absorbency do not fulfil all the needs of the users. For example some women may encounter the formation of unpleasant odour associated with milk discharge. Indeed in use, the pads -may acquire a variety of compounds, for example carbohydrates (like lactose), proteins (like casein, lactoferrin and lactoalbumin) and lipids (like triglycerides and fatty acids).

Malodourous compounds may be present in the milk discharge or may be developed by chemical reactions and/or any fluid degradation mechanisms once the fluid is absorbed into the pad. Milk discharge usually contain microorganisms and/or enzymes that can generate malodorous by products as a result of degradation mechanisms like putrefactive degradation, acid degradation, proteins degradation, fat degradation and the like. Unpleasant odours which emanate from the pads when in use may make the wearer feel self conscious.

Furthermore the micro-organisms present in the milk discharge may also cause infections or be associated with occurrence of skin problems on the breast. Pathogenic micro-organisms responsible of skin infections and/or skin irritation problems may belong to the family Ascomycetes, Pseudomonadaceae and Micrococcaceae and the genus Streptococcus, e.g., Candida albicans, Pseudomonas, Staphylococcus and Streptococcus.

It is a particular object of the present invention to provide breast pads which exhibit effective odour control over a wide range of malodor associated with milk discharge. More generally, it is an object of the present invention to provide breast pads suitable for application on the surface of breasts of nursing mothers, without permitting pathogenic micro-organisms to grow or to become active to an extent such as to promote undesirable odours, to incur the risk of infections and/or of negative effect on the skin. It is a further object of the present invention to meet the above mentioned needs while being safe to babies.

It has now been found that the above needs can be addressed by providing breast pads comprising lactic acid-producing micro-organisms. Indeed the presence of such lactic acid-producing micro-organisms in a breast pad provides effective antagonistic properties against undesirable strains of micro-organisms, typically present or arisen in the pad or on the surface (i.e., breast skin) the pad is applied to.

The antagonistic properties delivered by the microorganisms present in the breast pads herein will result in the reduction or even prevention of malodor formation as well as in reduction of the risk of skin infection and/or skin irritation. Besides these benefits the antagonistic properties of the microorganisms herein will also translate in the ability of the pads to disinfect the nipple area before feeding the baby.

The lactic acid producing microorganisms used herein have the ability to deliver antagonistic properties against undesirable pathogens which are known to cause unpleasant odors like for example the bacteria belonging to the family Enterobacteriaceae, e.g., Proteus mirabilis, Proteus vulgaris, Echerichia coli and Klebsiella. It is believed that the metabolic activity of such pathogen bacteria which aims for satisfying the bacteria needs for energy and proliferation, leads to degraded odorous compounds as by products which are among others low molecular weight fatty acids, amines, mercaptan, indoles, sulfide and the like. The antagonistic microorganisms according to the present invention inhibit the growth of such pathogen microorganisms, by competing for substrate and generating a non conducive acidic environment by changing the pH to more acidic values (e.g., pH 4 to 4.5). The antagonistic properties of the lactic-acid producing microorganisms according to the present invention are also partly denoted their ability of producing other antimetabolites, like enzymes (e.g., lactoperoxidases), toxins, carbon dioxide, peroxides or antibiotics, so-called bacteriocines.

Advantageously these benefits are delivered by being not only safe to babies but by having a positive impact on the heath of the babies. Indeed while sucking the nipple of his mother the baby may ingest such micro-organisms as a result of the presence of such micro-organisms on the nipple and/or skin of the breast of his mother after having been in contact with the pads herein. The occurrence of such an ingestion is believed to contribute to the natural equilibrium of micro-organisms in the gastric and intestinal tract of babies.

In a preferred embodiment the lactic-acid producing micro-organisms used herein are the spore-forming lactic acid-producing micro-organisms and more preferably the species *Lactobacillus sporogenes.* These micro-organisms have the ability to create very quickly an environment that is not suitable for the growth of pathogens. This is due to the rapid growth, high yield and reproducibility of such microoganisms in comparison to other lactic acid producing bacteria like *Lactobacillus acidophilus.* Furthermore these micro-organisms are spore forming micro-organisms, i.e., they are able to survive longer and reproduce themselves in comparison to non spore-forming micro-organisms. In other words, by the formation of spores these micro-organisms can germinate and re-germinate in time sequence in line with the milk discharge (growth media) into a breast pad, thereby ensuring long-lasting antagonistic properties against pathogens, typically long lasting odour control.

In the most preferred embodiment of the present invention the dormant form of such spore-forming lactic-acid producing micro-organisms, i.e., spores (preferably *L. sporogenes* spores) are used as the lactic acid producing micro-organisms herein. These spores have the ability to germinate in the living form of the micro-organisms (viable bacilli) and carry on their life activities, thereby exhibiting antagonistic properties against undesirable pathogens. The use of such spores in the pads herein provides improved stability both during storage and use. Indeed the use of the spores herein provides an effective odour controlling breast pad which can be stored for long periods of time before its actual use, without risking that the odour control ability of the pad in use is impaired. Actually, the use of these spores able storage stability upon prolonged periods of time up to the time the pad is used, i.e., is applied on the surface of a breast of a nursing mother where it typically comes into contact with milk discharge, and undergoes environmental changes that will create favourable environmental condition for the germination of the spores. Indeed the genetic identity, the lactic acid producing ability and the viability of the antagonistic microorganisms when using such spores are not impaired upon prolonged periods of storage and are maintained upon prolonged periods of use.

Advantageously, in the preferred embodiment herein wherein the spores as described herein are used the pads according to the present invention retain their whole antagonistic properties against pathogens up to the time their are used. Thus the present invention provides breast pads with effective antagonistic ability, while using reduced total amount of active material like odour control active.

A further advantage associated with the breast pads according to the present invention, is a better feeling and more acceptable cleanness level in use. Indeed, it has been found that the microorganisms herein allow gelification of the milk discharge, thereby facilitating the fluid transport into the pad. In a preferred embodiment the microorganisms are located in the core of the pad, thereby changing the physical properties of the milk discharge. Such gelification will have the benefits of reducing the rewetting of the topsheet and the soiling through. This translates in consumer noticeable dryness and cleanness benefit. Thus the present invention provides a breast pad which combines outstanding antagonistic properties like odor control over a broad spectrum of malodor and effective level of protection upon prolonged and/or abundant milk discharge.

### Summary of the Invention

The present invention relates to a breast pad comprising lactic acid producing micro-organisms. In a preferred embodiment herein the pads further comprise an absorbing gelling material.

### Brief description of the drawings

The invention is further described with reference to the accompanying drawing.

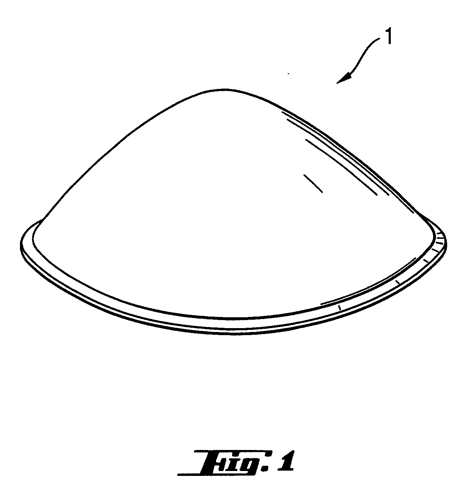

Fig. 1 shows a perspective view of breast-milk absorbent pad according to the invention.

### Detailed description of the Invention

The present invention relates to breast pads comprising lactic acid producing micro-organisms. According to the present invention the breast pads for use herein are disposable breast pads.

The term 'disposable' is used herein to describe breast pads which are not intended to be launched or otherwise restored or reused as a breast pad (i.e., they are intended to be discarded after a single use and, preferably to be recycled, composted or otherwise disposed of in an environmentally compatible manner).

By "milk discharge" it is meant herein any fluid produced by the breast of nursing mothers during the latter stages of pregnancy and after child birth. The present invention controls malodour associated with the occurrence of milk discharge but also those that might be associated with perspiration.

### The lactic acid producing micro-organisms

According to the present invention the breast pads comprise as an essential component a lactic acid producing microorganism or a mixture thereof.

Suitable lactic acid producing microorganisms for use herein also called antagonistic microorganisms are microorganisms that exhibit antagonistic properties against undesirable strain of microorganisms by releasing amongst other metabolites, lactic acid. The microorganisms that exhibit such antagonistic properties may be bacteria or other microorganisms for instance fungi.

Suitable lactic acid producing bacteria (antagonistic bacteria) for use herein include those belonging to the genera Lactobacillus, Lactococcus, Pedioccocus and/or Leuconostoc, and preferably the species *Lactobacillus acidophilus, Lactobacillus curvatus, Lactobacillus plantarum, Lactobacillus jenseni, Lactobacillus casei, Lactobacillus fermentum, Lactococcus lactis, Pedioccocus acidilacti, Pedioccocus pentosaceus, Pedioccocus urinae* and/or *Leuconostoc mesenteroides.*

Highly preferred lactic acid-producing microorganisms for use herein are the spore-forming lactic acid-producing microorganisms. These preferred microorganisms have the ability to survive in hostile environment in spore form (dormant form). Sporulation is the development in microorganisms of bodies each wrapped in a protective coat (a natural process of microencapsulation in a calcium-dipicolinic acid-peptidoglycan complex). Under favorable conditions, the spores germinate into viable bacilli (living form) and carry on their life activities. Suitable spore-forming lactic acid-producing microorganisms for use herein include the microorganisms belonging to the family Sporolactobacillaceae, particularly the genus Sporolactobacillus (e.g., S. *inulinus).* Highly preferred for use herein is the species *Lactobacillus sporogenes* (in its living and/or dormant form).

*L. sporogenes* was first isolated from green malt and described in 1933 by L.M. Horowitz-Wlassowa and N.W. Nowotelnow. It was submitted as L. *sporogenes* in the fifth edition (1939) of 'Bergey's manual of Determinative Bacteriology' as well as mentioned in recognized scientific publication, Korean J. Appl. Microb. & Bioengin. (1985) 13:185-190, J. Pharmaceut. Soc. Korea (1977) vol XXIII, 1-Feb, 473-474. *L. sporogenes* was transferred to *Bacillus coagulans* in the seventh edition of 'Bergey's manual of Determinative Bacteriology' due to simplification in cataloguing. However in honour of the original discoverers the name *L. sporogenes* is used widely. Reference is also made to the taxonomical classification of *Sporolactobacillus* in L'integratore Nutrizionale 2 (1) 1999, Stabilita' di integratori con Sporolactobacillus, classificazione tassonomica by L. Marossi and all.

According to the Eighth Edition of Bergey's Manual of Determinative Bacteriology, "various spore-bearing rods which produce lactic acid, are facultative or aerobic and catalase positive, have generally and correctly been assigned to the genus *Bacillus'.* The characteristics of L. *sporogenes* as cited in 'Bergey's Manual of Determinative Bacteriology' (seventh edition) and other sources are "gram positive spore-forming rods 0.9 by 3.0 to 5.0 micron size, aerobic to microaerophilic, producing L (+) lactic acid homofermentatively". *L. Sporogenes* also release other metabolites like carbon dioxide, diacetyl, bacteriocins, lacto-peroxidase.

Since *L. Sporogenes* exhibits characteristics typical of both genera *Lactobacillus* and *Bacillus,* its taxonomic position between the families *Lactobacillaceae* and *Bacillaceae* has often been discussed. This along with the fact that there is no universally accepted official classification leaves room for controversy in the nomenclature. More information about *L.sporogenes* is available from the commercial brochure 69/107, incorporated herein by reference, of Sochim International s.p.a. Milano, a supplier of the spore form of *L. Sporogenes.*

Some authors refer to *L.sporogenes* as *Bacillus coagulans. Bacillus coagulans* Hammer deposited as *Lactobacillus* sporogenes by Kabushiki Kaisha Naruse Fermentation Research Laboratory is commercially available under ATCC number 31284 (Internet information source : http://www.atcc.org/). ATCC stands for American Type Culture Collection.

*Lactobacillus sporogenes*, are preferred herein as they have the ability to create very quickly (faster than for example non-spore forming Lactobacillus) an environment that is not suitable for the growth of pathogens. This is due to the rapid growth, high yield and reproducibility of these micro-organisms in comparison to other lactic acid producing bacteria like *Lactobacillus acidophilus.* For example *L. sporogenes* needs 30 minutes for one generation while L. *acidophilus* needs 80 minutes. Furthermore these micro-organisms are spore forming lactic acid producing micro-organisms, i.e., they are able to survive longer and reproduce themselves in comparison to non spore-forming micro-organisms. Indeed in contrast to non-spore forming bacteria, L. *sporogenes* (living form) are transformed in spores (dormant form) when the substrate is reduced (in absence or reduced amount of milk discharge) and germinate again upon further milk discharge, i.e., reproduce themselves again through the spore form. In other words, by the formation of spores these micro-organisms can germinate and re-germinate in time sequence in line with the milk discharge (growth media) into a breast pad, thereby ensuring long-lasting antagonistic properties against pathogens, typically long lasting odour control.

It is understood herein that the lactic acid producing micro-organisms according to the present invention also include the spores (dormant form) of the micro-organisms (living form)when referring to *Sporolactobacillus* and *L. sporogenes.* These spores are typically activated due to the presence of substrate, temperature increase and pH change. The optimum growth temperature range for the spores of *L sporogenes* is between 30°C and 50°C and the optimum pH range is from 5.0 to 6.5.

*L.sporogenes* spores are ellipsoidal bodies measuring 0.9 to 1.2 by 1.0 to 1.7 microns. These spores are commercially available in a white to greyish powder form. *L. sporogenes* spores powder is commercially available under the name LACTOSPORE® from SABINSA CORPORATION (Sochim international s.p.a, Milano). 1 gram of LACTOSPORE® corresponds to 15^{*}10⁹ spores and thus to 15*10⁹·cfu (colony forming unit) of *L. sporogenes*.

In the embodiment of the present invention where the spores as described herein before are used, the breast pads, can be stored for longer periods of time before their actual use and still contain their whole antagonistic capacity up to the time the pads are used. *Indeed L. sporogenes* cells (in spore form) are protected from destruction by environmental factors by the naturally-present microencapsulation system, the spore coat. These spores are activated by environmental changes like pH and temperature changes and availability of substrate. For instance when the pad is contacted with the skin of the breast, the temperature increases (from room temperature to 30°C-35°C) and substrates like perspiration (pH 6), and/or milk discharge (pH 6.4 ) are provided. Such conditions will active the germination/re-germination of the spores. The spore coat imbibe water, swell and the increased water content will cause a rise in the metabolic rate of the sporulated bacilli. Outgrowths will begin to protrude from the spore-coats. The outgrown cells germinate and transform into viable vegetative cell (also called 'living form' herein). The living form begin to proliferate multiplying rapidly. These living forms continue their metabolic activities producing lactic acid and other metabolites which render the environmental non-conducive for the growth of harmful pathogenic micro-organisms. The germination process and more particularly the re-germination process (i.e., subsequent germination after the micro-organisms have been transformed into spores upon decrease of substrate availability) will be influenced by the milk discharge in the breast pad, leading to a somehow controlled germination/re-germination process on demand. In other words all the spores will not germinate with the same kinetic but the germination will be in relation to the milk discharge in the pad.

The lactic acid producing microorganisms (living form) are typically used in a freeze-dried form. Their isolation process follows known routine processes for the isolation of pure cultures. The isolated pure cultures are then typed according to known methods, e.g., API. The desired lactic acid producing bacteria are then cultivated in a fermentor in a manner known per se, are separated from the medium using a separator or a centrifuge, are freeze-dried in a manner known per se and ground to a fine powder. The bacterial concentrate in powder so obtained is then typically mixed with a fermentable carbohydrate, e.g., glucose, to a desired concentration. Such powder is then ready for use in a breast pad. Alternatively in some case it is possible to add the living bacteria to the breast pad and then carry out a freeze drying thereof. Another powder available form of the microorganisms herein is a lyophilized form.

Typically the number of lactic acid producing microorganisms per pad, exceeds 10²cfu, preferably exceeds 10⁴ cfu, more preferably exceeds 10⁶ cfu, and most preferably is between 10⁷ to 10¹⁰ cfu.

Pathogens bacteria that cause bad smell and/or skin problems may belong to, for example, the families Enterobacteriaceae, Ascomycetes, Pseudomonadaceae and Micrococcaceae and the genus Streptococcus. Examples of species and genera are Pseudomonas, Candida albicans, Escherichia coli, Proteus mirabilis, Proteus vulgaris, Enterococcus, Klebsiella, Staphylococcus and Streptococcus.

The particularities of the antagonistic microorganisms herein is that they inhibit other microorganisms by competing for substrates, forming metabolites like lactic acid, enzymes like lacto-peroxidases, toxins, carbon dioxide, peroxides or antibiotics, so-called bacteriocines. They have the ability to sustain the growth and reduce the patogenicity of many pathogens like ones mentioned herein before and especially Proteus, Pseudomonas, Echerichia, Klebsiella, Enterococcus, Staphylococcus, Streptococcus and/or Candida.

It is speculated that the production of lactic acid lowers the pH to 4-5, thereby inhibiting the growth of putrefactive organisms like E.coli, which require a higher optimum pH (typically 6 to 7) for favourable growth conditions. Furthermore undissociated lactic acid has the tendency to penetrate the membrane of pathogens, lowering their intracellular pH and/or interfering with their metabolic processes such as oxidative phosphorylation, thereby inhibiting the growth of such pathogens.

Other metabolites further contribute to inhibit the growth of pathogens. For example carbon dioxide is believed to reduce membrane permeability. Hydrogen peroxide / Lactoperoxidase are believed to oxidise basic proteins and to destroy the "enzymes factories" (ribosomes) of pathogens. Bacteriocins are proteins or protein complexes with bactericidal activity. Indeed, bacteriocins have the ability to link to particular receptors on the cell wall of microorganisms, thereby affecting the functionality of the cell wall/membranes. Bacteriocins are also able to affect DNA-synthesis and protein synthesis.

The particularity of the antagonistic microorganisms herein is that they are naturally occurring microorganisms that are non-toxic and do not have any negative biological effect on humans including babies.

One advantage afforded by the use of antagonistic microorganisms is that there is avoided an undesired selection pressure on the micro environment, such as favoring potential desease-promoting microorganisms and therewith the risk of developing pathogenic strains that are resistant to antibiotics and chemopharmaceutical preparations. Since the antimicrobial system is based on a natural, biological process, there is less risk of environmental ecological and toxic disturbances.

Advantageously beside the antagonistic properties of the microorganisms used herein it has further been observed that these microorganisms have the advantage of changing the physical properties of milk fluid. Indeed a gelification of the milk discharge is obtained upon contact with the microorganisms herein. This results in reduced leakage/wet through of the pads as well as in improved dryness of the wearer facing surface. When talking about reduced leakage and/or improved dryness reference is made to a same breast pad in absence of such microorganisms. As used herein the tospheet provides said wearer facing surface. Improved dryness and cleanness is particularly noticed on the wearer facing surface in those embodiments wherein the lactic acid producing microorganisms are located in the absorbent core.

Advantageously the present invention provides breast pads which upon contact with milk discharge, provide effective and long lasting antagonistic properties, especially odor control properties, but also effective level of protection of the pads, resulting in consumer noticeable benefits like dryness and cleanness.

Without to be bound by theory, it is speculated that the cause of this gelification is the denaturation of the proteins. Indeed the lactic acid-producing microorganisms herein, through the glycogen fermentation, produce lactic acid. As proteins are sensitive to pH, the presence of lactic acid causes the soluble proteins contained into milk discharge to turn into insoluble form. This creates a sort of tri-dimensional net of molecules trapping globules, minerals, fats which results in the so called gelification of the milk discharge.

### Optional agents

The pads according to the present invention may further comprise other conventional agents like absorbent gelling materials and/or additional odor control agents.

### Absorbent gelling materials

As is well-known from recent commercial practice, absorbent gelling materials (sometimes referred to as "super-sorbers") are becoming broadly used in absorbent articles. AGM's are materials which have the twofold property of absorbing and retaining fluids and odours, and thus further contribute to the benefit of the present invention.

Such materials form hydrogels on contact with water (e.g., with urine, blood, and the like). One highly preferred type of hydrogel-forming, absorbent gelling material is based on polyacids, especially polyacrylic acid. Hydrogel- forming polymeric materials of this type are those which, upon contact with fluids (i.e., liquids) such as water or body fluids, imbibe such fluids and thereby form hydrogels. These preferred absorbent gelling materials will generally comprise substantially water-insoluble, slightly cross-linked, partially neutralized, hydrogel-forming polymer materials prepared from polymerizable, unsaturated, acid-containing monomers. In such materials, the polymeric component formed from unsaturated, acid-containing monomers may comprise the entire gelling agent or may be grafted onto other types of polymer moieties such as starch or cellulose. Acrylic acid grafted starch materials are of this latter type. Thus, the preferred absorbent gelling materials include hydrolyzed acrylonitrile grafted starch, acrylic acid grafted starch, polyacrylates, maleic anhydride-based copolymers and combinations thereof. Especially preferred absorbent gelling materials are the polyacrylates and acrylic acid grafted starch.

Whatever the nature of the polymer components of the preferred absorbent gelling materials, such materials will in general be slightly cross-linked. Crosslinking serves to render these preferred hydrogel-forming absorbent materials substantially water-insoluble, and cross-linking also in part determines the gel volume and extractable polymer characteristics of the hydrogels formed therefrom. Suitable cross-linking agents are well known in the art and include, for example, (1) compounds having at least two polymerizable double bonds; (2) compounds having at least one polymerizable double bond and at least one functional group reactive with the acid-containing monomer material; (3) compounds having at least two functional groups reactive with the acid-containing monomer materials; and (4) polyvalent metal compounds which can from ionic cross-linkages. Cross-linking agents of the foregoing types are described in greater detail in Masuda et al; U.S. Patent 4,076,663; Issued February 28, 1978. Preferred cross-linking agents are the di- or polyesters of unsaturated mono-or polycarboxylic acids with polyols, the bisacrylamides and the di-or triallyl amines. Especially preferred cross-linking agents are N,N'-methylenebisacrylamide, trimethylol propane triacrylate and triallyl amine. The cross-linking agent will generally comprise from about 0.001 mole percent to 5 mole percent of the preferred materials. More preferably, the cross-linking agent will comprise from about 0.01 mole percent to 3 mole percent of the gelling materials used herein.

The preferred, slightly cross-linked, hydrogel-forming absorbent gelling materials will generally be employed in their partially neutralized form. For purposes described herein, such materials are considered partially neutralized when at least 25 mole percent, and preferably at least 50 mole percent of monomers used to form the polymer are acid group-containing monomers which have been neutralized with a salt-forming cation. Suitable salt-forming cations include alkali metal, ammonium, substituted ammonium and amines. This percentage of the total monomers utilized which are neutralized acid group-containing monomers is referred to as the "degree of neutralization". Typically, commercial absorbent gelling materials have a degree of neutralization somewhat less than 90%.

The preferred absorbent gelling materials used herein are those which have a relatively high capacity for imbibing fluids encountered in the absorbent articles; this capacity can be quantified by referencing the "gel volume" of said absorbent gelling materials. Gel volume can be defined in terms of the amount of synthetic urine absorbed by any given absorbent gelling agent buffer and is specified as grams of synthetic urine per gram of gelling agent.

Gel volume in synthetic urine (see Brandt, et al, below) can be determined by forming a suspension of about 0.1-0.2 parts of dried absorbent gelling material to be tested with about 20 parts of synthetic urine. This suspension is maintained at ambient temperature under gentle stirring for about 1 hour so that swelling equilibrium is attained. The gel volume (grams of synthetic urine per gram of absorbent gelling material) is then calculated from the weight fraction of the gelling agent in the suspension and the ratio of the liquid volume excluded from the formed hydrogel to the total volume of the suspension. The preferred absorbent gelling materials useful in this invention will have a gel volume of from about 20 to 70 grams, more preferably from about 30 to 60 grams, of synthetic urine per gram of absorbent gelling material.

Another feature of the most highly preferred absorbent gelling materials relates to the level of extractable polymer material present in said materials. Extractable polymer levels can be determined by contacting a sample of preferred absorbent gelling material with a synthetic urine solution for the substantial period of time (e.g., at least 16 hours) which is needed to reach extraction equilibrium, by then filtering the formed hydrogel from the supernatant liquid, and finally by then determining the polymer content of the filtrate. The particular procedure used to determine extractable polymer content of the preferred absorbent gelling agent buffers herein is set forth in Brandt, Goldman and Inglin; U.S. Patent 4,654,039; Issues March 31,1987, Reissue 32,649, The absorbent gelling materials which are especially useful in the pads herein are those which have an equilibrium extractables content in synthetic urine of no more than about 17%, preferably no more than about 10% by weight of the absorbent gelling material.

The absorbent gelling materials herein before described are typically used in the form of discrete particles. Such absorbent gelling materials can be of any desired shape, e.g., spherical or semi-spherical, cubic and the like.

The size of the absorbent gelling material particles may vary over a wide range. For reason of industrial hygiene, average particle sizes smaller than about 30 microns are less desirable. Particles having a smallest dimension larger than about 2mm may also cause a feeling of grittyness in the pad, which is undesirable from a consumer aesthetics standpoint. Furthermore, rate of fluid absorption can be affected by particle size. Larger particles have very much reduced rates of absorption. Preferred for use herein are absorbent gelling materials particles substantially all of which have a particle size of from about 30 microns to about 2mm. "Particle Size" as used herein means the weighted average of the smallest dimension of the individual particles.

Typically, the breast pads herein may comprise absorbent gelling material particles at levels ranging from 1 gm⁻² to 300gm⁻², preferably from 30gm⁻² to 150gm⁻², more preferably from 55gm⁻² to 85gm⁻².

### Odour control agents

For instance additional odour control agent or combinations thereof, known in the art for this purpose may be used herein. These agents can typically be classified according to the type of odour the agent is intended to combat. Odors may be chemically classified as being acidic, basic or neutral.

Alternatively, the odor control agents may be categorized with respect to the mechanism by which the malodor detection is reduced or prevented. For example, odor control agents which chemically react with malodorous compounds or with compounds which produce malodorous degradation products thereby generating compounds lacking odor or having an odor acceptable to consumers may also be utilized herein.

Suitable odor control agents for use herein typically include carbonates (e.g., sodium carbonate), bicarbonates (e.g., sodium bicarbonate), phosphates (e.g., sodium phosphate), sulphates (e.g., zinc and copper sulphates), carboxylic acids such as citric acid, lauric acid, boric acid, adipic acid and maleic acid, activated carbons, clays, zeolites, silicas and starches. Such odor control agents and systems are disclosed in more details hereinafter and for example in EP-A-348 978, EP-A- 510 619, WO 91/12029, WO 91/11977, WO 91/12030, WO 81/01643 and WO 96/06589.

Alternative odor control agents are ion exchange resins such as those described in US 4 289 513 and US 3340875.

Masking agents such as perfumes may also be used as odor control agents herein.

Typically, the breast pads may comprise the additional odor control agent or a mixture thereof at a level of from 0 gm⁻² to 600 gm⁻², preferably from 5 to 500 gm⁻², more preferably from 10 gm⁻² to 350 gm⁻² and most preferably from 20 gm⁻² to 200 gm⁻²

### The breast pads

The breast pads according to the present invention typically comprise a liquid pervious topsheet, a backsheet and an absorbent core intermediate said backsheet and said topsheet.

Suitable breast pads for use herein may have any configuration from circular to non-circular so as to be adapted to the natural morphology of women breasts. Typically breast pads for use herein are flat disk shaped pads or conic-shaped pads.

Example of conic-shaped breast pads according to the present invention are described in for example US 843 062, US 5 690 536, US 5 017 174, US 4 700 699, US 5683 286 and/or EP 698 385, all incorporated herein by reference.

Particularly suitable breast pads for use herein have a topsheet, a backsheet and an absorbent core intermediate the topsheet and the backsheet, and are typically as described in the following description:

### Absorbent core

According to the present invention, the absorbent core may include the following components: (a) an optional primary fluid distribution layer preferably together with a secondary optional fluid distribution layer; (b) a fluid storage layer; and (c) an optional fibrous ("dusting") layer underlying the storage layer. According to the present invention the absorbent may have any thickness depending on the end use envisioned.

### a Primary/Secondary Fluid Distribution Layer

One optional component of the absorbent 'according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized. The fluid distribution layers can be comprised of any material typical for such distribution layers. In particular fibrous layers maintain the capillaries between fibers even when wet are useful as distribution layers.

### b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials in combination with suitable carriers.

Suitable carriers include materials which are conventionally utilized in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations.

An embodiment of the absorbent structure made according to the present invention may comprise multiple layers comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orion), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

### c Optional Fibrous ("Dusting") Layer

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core.

### The topsheet

According to the present invention the breast pads comprise as an essential component a topsheet. The topsheet may comprise a single layer or a multiplicity of layers. In a preferred embodiment the topsheet comprises a first layer which provides the user facing surface of the topsheet and a second layer between the first layer and the absorbent structure/core.

The topsheet as a whole and hence each layer individually needs to be compliant, soft feeling, and non-irritating to the wearer's skin. According to the present invention the topsheet may be formed from any of the materials available for this purpose and known in the art, such as woven and non woven fabrics and films.

In a preferred embodiment of the present invention at least one of the layers, preferably the upper layer, of the topsheet comprises a hydrophobic, liquid permeable apertured polymeric film. Preferably, the upper layer is provided by a film material having apertures which are provided to facilitate liquid transport from the wearer facing surface towards the absorbent structure. If present the lower layer preferably comprises a non woven layer, an apertured formed film or an airlaid tissue.

The term apertured polymeric topsheet as used herein refers to topsheets comprising at least one layer or a multiplicity of layers wherein at least one layer is formed from a continuous or uninterrupted film material wherein apertures are created. It has been surprisingly discovered that apertured polymeric film topsheets yield significantly better odor control than other types of topsheets such as for example thermal bonded nonwoven materials.

In general the apertured polymeric topsheet of the present invention is compliant, soft feeling, and non-irritating to the wearer's skin. Further, the topsheet is fluid pervious permitting fluids (e.g., milk) to readily penetrate through its thickness. Suitable apertured polymeric film topsheets for use herein include polymeric apertured formed films, thermoplastic films, apertured plastic films, and hydroformed thermoplastic films; porous foams; net- like reticulated foams and thermoplastic films; and thermoplastic scrims.

### The backsheet

The backsheet primarily prevents the extrudes absorbed and contained in the absorbent structure from wetting articles that contact the breast pad. The backsheet is preferably impervious to liquids (e.g. milk) and is preferably manufactured from a thin plastic film, although other liquid impervious materials can also be used.

The backsheet can comprise a woven or nonwoven material, polymeric films such as thermoplastic films of polyethylene or polypropylene, or composite materials such as a film-coated nonwoven material. Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385.

The microorganisms as described herein and optional conventional agents (AGM and additional odor control agent) may be incorporated into the breast pad by any of the methods disclosed in the art, for example layered on the core of the pad or mixed within the fibres of the absorbent core.

The microorganisms as described herein and the optional agents are preferably incorporated between two layers of cellulose tissue. Optionally the system may be bonded between two cellulose tissue layers with, for example, a hot melt adhesive (polyethylene powder) or any suitable bonding system, as described in WO 94/01069.

Polyethylene powder may be replaced by a conventional glue for instance those commercially available from ATO Findley under the name H20-31® to glue the laminate layers and/or components together. Advantageously this method step allows to avoid the heating step necessary when using polyethylene powder.

The microorganisms and optional agents may be distributed homogeneously or non homogeneously over the entire absorbent structure or in at least one layer of the topsheet or in at least one layer of the core or any mixture thereof. The microorganisms and the optional agents may be distributed homogeneously or non homogeneously on the whole surface of the'desired layer or layers, or on one or several area of the surface layer/layers to which it is positioned (e.g. central area and/or surrounding area like the edges of a layer of the pad) or mixtures thereof. Preferably the microorganisms herein are located in the core (also called intermediate layer which is positioned between the topsheet and the backsheet). The presence of the microorganisms in the core is preferred because the core collects and absorbs the milk discharge. Thus the close proximity to the substrate contributes to improved antagonistic activity of the microorganisms. Indeed optimum inhibition of the degradation activity by putrefactive bacteria and proliferation of pathogens is obtained.

The microorganisms as described herein and the optional agents may be independently incorporated as a powder, typically when the spore form is used like Lactospore® and when the living form is used in lyophilized form or freeze-dried. When used in a granulate or particulate form the microorganisms powder as described herein, the optional odor absorbing agents and optional absorbing gelling material may be granulated separately and then mixed together or granulated together.

## Claims

1. A breast pad comprising lactic acid producing microorganisms.

2. A pad according to claim 1, wherein said lactic acid producing microorganisms belong to the genera Lactobacillus, Lactococcus, Pedioccocus, Leuconostoc and/or Sporolactobacillus and more preferably to the species *Lactobacillus sporogenes*, *Lactobacillus acidophilus, Lactobacillus curvatus, Lactobacillus plantarum, Lactobacillus jenseni, Lactobacillus casei, Lactobacillus fermentum, Lactococcus lactis, Pediccocus acidilacti, Pediccocus pentosaceus, Pediccocus urinae, Leuconostoc mesenteroides and/or Sporolactobacillus inulinus.*

3. A pad according to any of the preceding claims wherein the lactic acid producing microorganisms are spore-forming lactic acid producing microorganisms, preferably the species *Lactobacillus sporogenes* in their living form or dormant form (spores).

4. A pad according to any of the preceding claims which comprises more than 10²cfu, preferably more than 10⁴ cfu, even more preferably more than 10⁶ cfu, and most preferably between 10⁷ to 10¹⁰ cfu of lactic acid producing microorganisms.

5. A pad according to any of the preceding claims which further comprises an absorbent gelling material or a mixture thereof.

6. A pad according to claim 5, wherein the absorbent gelling material or a mixture thereof is present at a level from 1gm⁻² to 300gm⁻², preferably from 30gm⁻² to 150gm⁻², more preferably from 55gm⁻² to 85gm⁻²

7. A pad according to any of the preceding claims wherein said pad comprises a liquid pervious topsheet, a backsheet and an absorbent core intermediate said backsheet and said topsheet.

8. A pad according to claim 7 wherein the microorganisms are disposed in the absorbent core
